(19) Europäisches Patentamt
European Patent Office
Office européen des brevets

(11) Veröffentlichungsnummer: **0 367 365**
**A2**

(12) # EUROPÄISCHE PATENTANMELDUNG

(21) Anmeldenummer: **89250071.1**

(51) Int. Cl.5: **C07D 275/04 , A01N 43/80**

(22) Anmeldetag: **31.10.89**

(30) Priorität: **03.11.88 DE 3837578**

(43) Veröffentlichungstag der Anmeldung:
**09.05.90 Patentblatt 90/19**

(84) Benannte Vertragsstaaten:
**AT BE CH DE ES FR GB GR IT LI LU NL SE**

(71) Anmelder: **SCHERING AKTIENGESELLSCHAFT**
**Müllerstrasse 170/78**
**D-1000 Berlin 65(DE)**

(72) Erfinder: **Hübl, Dieter, Dr.**
**Gerhard-Hauptmannstrasse 21**
**D-4709 Bergkamen(DE)**
Erfinder: **Pieroh, Ernst**
**Im Fischgrund 18**
**D-1000 Berlin 28(DE)**

(54) **Benzoisothiazole, Verfahren zu ihrer Herstellung und Verwendung als Schädlingsbekämpfungsmittel.**

(57) Es werden neue Benzoisothiazole der allgemeinen Formel I

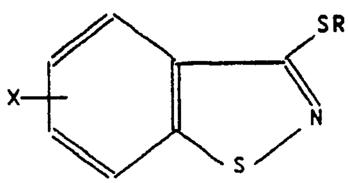

(I),

worin
X und R die in der Beschreibung angegebene Bedeutung haben, Verfahren zu ihrer Herstellung und ih Verwendung als Schädlingsbekämpfungsmittel, insbesondere gegen Nematoden, beschrieben.

EP 0 367 365 A2

## Benzoisothiazole, Verfahren zu ihrer Herstellung und Verwendung als Schädlingsbekämpampfungsm tel

Die Erfindung betrifft neue Benzoisothiazole, ihre Herstellung und Verwendung als Schädlingsbekäm fungsmittel, insbesondere gegen Nematoden.

Es sind bereits 3-Thio-5-nitro-benzisothiazole bekannt, die bakterielle und stark fungizide Eigenschaft haben (DE 3 202 298).

Aufgabe der vorliegenden Erfindung war es, Schädlingsbekämpfungsmittel mit besseren Eigenschaft bereitzustellen.

Es wurde nun gefunden, daß Verbindungen der allgemeinen Formel I

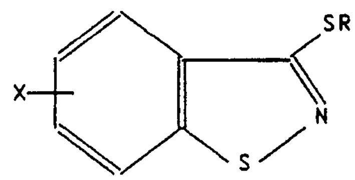

(I),

worin

X Wasserstoff, Halogen, $C_{1-4}$-Alkyl, $C_{1-4}$-Alkylthio, $C_{1-4}$-Alkoxy, Di-$C_{1-4}$-alkylamino, Halogen-$C_{1-4}$-alk Halogen-$C_{1-4}$-alkoxy, Halogen-$C_{1-4}$-alkylthio, $C_{3-6}$-cycloalkyl, Halogen-$C_{3-6}$-cycloalkyl, Halogen-$C_{3-6}$-c cloalkylmethoxy, Halogen-$C_{3-6}$-Cycloalkylmethylthio, Nitro, Cyano, Amino, Phenyl, Halogenphenyl, Ph noxy, Phenylthio oder Halogenphenylthio und

R einen ein- oder mehrfach, gleich oder verschieden durch Halogen substituierten $C_{1-12}$-Alkyl-, $C_{2-}$ Alkenyl-, $C_{2-12}$-Alkinyl-, $C_{3-6}$-Cycloalkyl-oder $C_{3-6}$-Cycloalkylmethylrest bedeuten,

eine überraschend gute nematizide Wirkung bei gleichzeitig guter Pflanzenverträglichkeit zeigen.

Unter Halogen ist Cl, F, Br und Jod zu verstehen.

Die Bezeichnung Halogenalkyl, Halogenalkoxy, Halogenalkylthio, Halogencycloalkyl besagt, daß e oder mehrere Wasserstoffatome des Alkyl- bzw. Cycloalkylrestes durch Halogen ersetzt sind.

Die erfindungsgemäßen Verbindungen der Formel I lassen sich nach an sich bekannten Method dadurch herstellen, daß man Verbindungen der Formel II

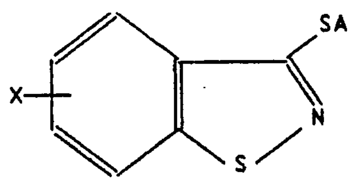

(II),

wobei X die in Formel I angegebene Bedeutung hat und A Wasserstoff, Ammonium oder ein Alkalime bedeutet, mit Verbindungen der Formel Z-R, wobei Z eine Fluchtgruppe wie z.B. Halogen, Mesylat u Tosylat ist und R die in Formel I angegebene Bedeutung hat, in einem inerten Lösungsmittel oc Lösungsmittelgemisch bei gebenenfalls erhöhter Temperatur und gegebenenfalls bei erhöhtem Druck Gegenwart einer Base umsetzt.

Als Basen können organische und anorganische Basen eingesetzt werden, wie z.B. tertiäre Amine v Triethylamin und Tripropylamin, Alkali- und Erdalkalimetall-hydride, -hydroxide, -carbonate und bicarbonate, aber auch Alkalialkoholate, wie z.ß. Natriummethylat oder Kalium-tert.-butylat.

Für die Herstellung der erfindungsgemäßen Verbindungen geeignete Lösungsmittel sind z.B. Diethy ther, Dioxan und Tetrahydrofuran, aliphatische und aromatische Kohlenwasserstoffe wie Toluol und Petrc ther; halogenierte Kohlenwasserstoffe wie Chlorbenzol, Methylenchlorid, Tetrachlorkohlenstoff, Chlorofoı Nitrile wie Acetonitril, Propionitril; N,H-dialkylierte Amide wie Dimethylformamid; Ketone wie Acet Methylethylketon: Dimethylsulfoxid, Sulfolan sowie Wasser und Alkohole, z.B. Methanol, Ethanol, Isoprop ol oder Butanol und die Gemische solcher Lösungsmittel untereinander.

Die Reaktion wird in Abhängigkeit von den Reaktanden bei Temperaturen zwischen -70 °C bis 120 durchgeführt. Auch in Abhängigkeit von den Reaktanden kann die Anwendung von Druck zweckmäßig se Der dann ange wendete Druck liegt im Bereich zwischen 1 bis 25 bar. Die Reaktionsdauer beträgt ca. · bis 48 Stunden. Das Reaktionsgemisch wird anschließend auf Eis/Wasser gegossen, extrahiert und in

sich bekannter Weise aufgearbeitet. Die anfallenden Produkte lassen sich in üblicher Weise durc Umkristallisation, Vakuumdestillation oder Säulenchromatographie reinigen.

Die als Ausgangsmaterial verwendeten Verbindungen der Formel II sind entweder bekannt oder lasse sich nach an sich bekannten Verfahren herstellen.

Aufgrund der nematiziden Wirksamkeit bei guter Pflanzenverträglichkeit können die erfindungsgemäße Verbindungen mit Erfolg im Pflanzenschutz als Schädlingsbekämpfungsmittel in der Landwirtschaft, i Wein-, Obst- und Gartenbau und in Forstkulturen eingesetzt werden.

Zu den pflanzenparasitären Nematoden, die erfindungsgemäß bekämpft werden können, gehöre beispielsweise die Wurzelgallen-Nematoden, wie Meloidogyne incognita, Meloidogyne hapla, Meloidogyr javanica, die Zysten bildenden Nematoden, wie Globodera rostochiensis, Heterodera schachtii, Heterode avenae, Heterodera glycines, Heterodera trifolii, Stock- und Blattälchen, wie Ditylenchus dipsaci, Dityle chus destructor, Aphelenchoides ritzemabosi, Pratylenchus neglectus, Pratylenchus penetrans, Pratylench curvitatus sowie Tylenchorhynchus dubius, Tylenchorhynchus claytoni, Rotylenchus robustus, Heliocotyle chus multicinctus, Radopholus similis, Belonolaimus longicaudatus, Longidorus elongatus und Trichodor primitivus.

Aufgrund der insektiziden und akariziden Eigenschaften der erfindungsgemäßen Verbindungen biete sie darüberhinaus Anwendungsmöglichkeiten sowohl in der Behandlung gegen Schädlinge in den verschi densten Stadien der Kulturpflanzen als auch gegen Schädlinge an Mensch und Tier.

Die Anwendung der erfindungsgemäßen Wirkstoffe erfolgt in Form ihrer handelsüblichen Formulieru gen und/oder aus diesen Formulierungen bereiteten Anwendungsformen.

Der Wirkstoffgehalt der aus den handelsüblichen Formulierungen bereiteten Anwendungsformen kann weiten Bereichen variieren. Die Aufwandmenge für die Bekämpfung von Nematoden pro Hektar betrá etwa 0,03 kg bis etwa 10 kg, vorzugsweise etwa 0,3 kg bis etwa 6 kg.

Die Wirkstoffe oder deren Mischungen können in die üblichen Formulierungen überführt werden w Lösungen, Emulsionen, Spritzpulver, Suspensionen, Pulver, Stäubemittel, Schäume, Pasten, lösliche Pulve Granulate, Aerosole, Suspensions-Emulsionskonzentrate, Saatgutpuder, Wirkstoff-imprägnierte Natur- ur synthetische Stoffe, Feinstverkapselungen in polymeren Stoffen und in Hüllmassen für Saatgut, ferner Formulierungen mit Brennsätzen, wie Räucherpatronen, -dosen, -spiralen u.ä. sowie ULV-Kalt- ur Warmnebel-Formulierungen.

Diese Formulierungen werden in bekannter Weise hergestellt, z.B. durch Vermischen der Wirkstoffe m Streckmitteln, also flüssigen Lösungsmitteln, unter Druck stehenden verflüssigten Gasen und/oder feste Trägerstoffen, gegebenenfalls unter Verwendung von oberflächenaktiven Mitteln, also Emulgiermitte und/oder Dispergiermitteln und/oder schaumerzeugenden Mitteln.

Im Falle der Benutzung von Wasser als Streckmittel können z.B. auch organische Lösungsmittel a Hilfslösungsmittel verwendet werden.

Als flüssige Lösungsmittel kommen im wesentlichen in Frage aromatische Kohlenwasserstoffe w Xylol, Toluol oder Alkylnaphthaline, chlorierte Aromaten oder chlorierte aliphatische Kohlenwasserstoffe w Chlorbenzole, Chlorethylene oder Methylenchlorid, aliphatische Kohlenwasserstoffe wie Cyclohexan od Paraffine, z.B. Erdölfraktionen, Alkohole wie Butanol, Glycol sowie deren Ether und Ester, Ketone w Aceton, Methylethylketon, Methylisobutylketon oder Cyclohexanon, stark polare Lösungsmittel wie Dim thylformamid und Dimethylsulfoxid sowie Wasser.

Mit verflüssigten gasförmigen Streckmitteln oder Trägerstoffen sind solche Flüssigkeiten gemei welche bei normaler Temperatur und unter Normaldruck gasförmig sind, z.B. Aerosol-Treibgase w Halogenkohlenwasserstoffe sowie Butan, Propan, Stickstoff und Kohlendioxid.

Als feste Trägerstoffe kommen infrage natürliche Gesteinsmehle wie Kaoline, Tonerden, Talkur Kreide, Quarz, Attapulgit, Montmorillonit oder Diatomeenerde und synthetische Gesteinsmehle wie hochdi perse Kieselsäure, Aluminiumoxid und Silikate sowie als feste Trägerstoffe für Granulate gebrochene ur fraktionierte natürliche Gesteine wie Calcit, Marmor, Bims, Sepiolith, Dolomit sowie synthetische Granula aus anorganischen und organischen Mehlen sowie Granulate aus organischem Material wie Sägemel Kokosnußschalen, Maiskolben und Tabakstengel.

Als Emulgatoren bzw. schaumerzeugende Mittel seien genannt nicht-ionogene und anionische Emulg toren wie Polyoxyethylen-Fettsäure-Ester, Polyoxyethylen-Fettalkohol-Ether, z.B. Alkylaryl-polyglykolethe Alkylsulfonate, Arylsulfonate sowie Eiweißhydrolysate.

Als Dispergiermittel seien z.B. Lignin, Sulfitablaugen und Methylcellulose genannt.

Es können in den Formulierungen auch Haftmittel wie Carboxymethylcellulose, natürliche und synthe sche pulvrige, körnige oder latexförmige Polymere verwendet werden wie Gummi arabicum, Polyvinylalk hol und Polyvinylacetat.

Weiterhin können Farbstoffe wie anorganische Pigmente, z.B. Eisendioxid, Titanoxid, Ferrocyanblau ur

organische Farbstoffe wie Alizarin-, Azo-Metallphthalocyaninfarbstoffe und Spurennährstoffe wie Salze v Eisen, Mangan, Bor, Kupfer, Kobalt, Molybdän und Zink mitverwendet werden.

Die Formulierungen enthalten im allgemeinen zwischen 0,1 und 95 Gewichtsprozent Wirkstoff, vorzug weise zwischen 0,5 und 90 %.

Beispiele für Formulierungen sind:

I. Spritzpulver

10 Gewichtsteile der Verbindung gemäß Beispiel 1 werden mit 12 Gewichtsteilen Calciumsalz der Lignins fonsäure. 76 Gewichtsteilen Kaolin und 2 Gewichtsteilen Dialkylnaphthalinsulfonat innig vermischt u. vermahlen.

II. Stäubepulver

2,5 Gewichtsteile der Verbindung gemäß Beispiel 1 werden in 10 Teilen Methylenchlorid gelöst und a eine Mischung von 25 Gewichtsteilen pulverförmige Kieselsäure und 71,5 Gewichtsteilen Talkum sowie Gewichtsteil Sudanrot gegeben. Das Lösungsmittel wird im Vakuum entfernt und der Rückstand fe vermahlen.

III. Granulat

5 Gewichtsteile der Verbindung gemäß Beispiel 1 werden in 10 Teilen Methylenchlorid gelöst und auf Gewichtsteile granuliertes Attapulgit der Korngröße 0,3 - 0,8 mm aufgesprüht und getrocknet.

IV. Emulsionskonzentrat 20 Gewichtsteile der Verbindung gemäß Beispiel 1 werden in einer M schung aus 75 Gewichtsteilen Isophoron und 5 Gewichtsteilen eines Gemisches aus 30 Teil Phenylsulfonat-Calciumsalz, 30 Teilen Rhizinus-Polyglycolat mit 40 Mol% Ethylenoxid und 40 Teilen ein Copolymeren von Propylen- und Ethylenoxid gelöst.

Die nachfolgenden Beispiele beschreiben die Herstellung der erfindungsgemäßen Verbindungen.

## Beispiel 1

3-Difluormethylthio-1,2-benzisothiazol

4,1 g (0,02 mol) 1,2-Benzisothiazol-3-thiolat, Kaliumsalz werden in 25 ml Dioxan suspendiert und r einer Lösung von 5,6 g (0,10 mol) Kaliumhydroxid in 9 ml Wasser versetzt. Bei einer Temperatur von 60- °C wird 30 Minuten lang ein gleichmäßiger Strom von Chlordifluormethan eingeleitet. Man läßt abkühle gießt auf 500 ml Eiswasser und extrahiert mehrmals mit Essigester. Die organische Phase wird üb Magnesiumsulfat getrocknet, filtriert und eingeengt. Das verbleibende Öl wird an Kieselgel chromatogı phiert (Eluent Hexan/Diethylester 9:1).

Ausbeute: 1,0 g ≙ 22,9 % der Theorie

$n_D^{20}$ : 1,59912

## Beispiel 2

3-(3,4,4-Trifluor-3-butenylthio)-1 ,2-benzisothiazol

2,05 9 (0,01 mol) 1,2-Benzisothiazol-3-thiolat, Kaliumsalz werden in 25 ml Dimethylformamid suspe diert. Bei einer Temperatur von 20 °C wird eine Lösung von 2,3 (0,01 mol) 4-Brom-1,1,2-trifluorobuten-1 10 ml Dimethylformamid zugetropft. Es wird 2 Stunden bei Raumtemperatur nachgerührt. Das Reaktionsg misch wird auf 300 ml Eiswasser gegeben und mehrmals mit Essigester extrahiert. Die organische Pha wird über Magnesiumsulfat getrocknet, filtriert und eingeengt. Das verbleibende Öl wird an Kieselg chromatographiert (Eluent Hexan/Diethylester 9:1).

Ausbeute: 0,60 g ≙ 22 % der Theorie

$n_D^{20}$ : 1,58008

In analoger Weise werden die folgenden Verbindungen der Formel I hergestellt:

| Beispiel Nr. | X | R | $n_D^{20}$ / | Fp. (°C) |
|---|---|---|---|---|
| 3 | H | $-CF_2CF_2Br$ | 1,56868 | |
| 4 | H | 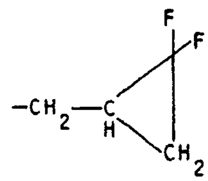 | 1,59688 | |
| 5 | H | $-CF_2Br$ | 1,62668 | |
| 6 | H | $-CH_2CH_2F$ | 1,62330 | |
| 7 | $5-NO_2$ | $-CHF_2$ | | 74-76 |
| 8 | $5-NO_2$ | $-CH_2CH_2CF=CF_2$ | | 69-71 |
| 9 | 6-Cl | $-CH_2CH_2CF=CF_2$ | 1,60740 | |
| 10 | 6-F | $-CH_2CH_2CF=CF_2$ | 1,54716 | |
| 11 | $6-CF_3$ | $-CH_2CH_2CF=CF_2$ | 1,53282 | |
| 12 | $5-NO_2$ | $-CF_2Br$ | | 74-76 |

Das nachfolgende Anwendungsbeispiel zeigt die biologische Wirkung der erfindungsgmäßen Verbi dungen.

## Anwendungsbeispiel A

Bekämpfung von Wurzelgallennematoden (Meloidogyne incognita)

10 %ige pulverförmige Wirkstoffzübereitungen werden gleichmäßig mit Boden vermischt, der mit de Testnematoden stark verseucht ist. Danach füllt man den so behandelten Boden in 0,5 Liter fassenc Tonschalen, sät Gurkensamen ein und kultiviert bei einer Bodentemperatur von 25-27 °C im Gewächshau Nach einer Kulturdauer von 25-28 Tagen werden die Gurkenwurzeln ausgewaschen, im Wasserbad a Nematodenbefall (Wurzelgallen) untersucht und der Wirkungsgrad des Wirkstoffes im Vergleich zur ve seuchten Kontrolle in % bestimmt. Wird der Nematodenbefall vollständig vermieden, wird der Wirkungsgra als 100 % festgesetzt.

Bei einer Dosis von 10 mg Wirkstoff je Liter Boden konnte ein Nematodenbefall durch Meloidogy incognita durch die Verbindung gemäß Beispiel 2 vollständig (100 %ig) vermieden werden.

## Anwendungsbeispiel B

Wirkung der prophylaktischen Futterbehandlung gegen die Gemeine Bohnen-Spinnmilbe (Tetranych urticae Koch)

Aus entwickelten Primärblättern der Buschbohne (Phaseolus vulgaris nanus Aschers.) werden run Blattscheiben mit einem Durchmesser von 14 mm gestanzt und unbehandelt bzw. nach Tauchbehandlu mit einer 0,1 % Wirkstoff enthaltenden wäßrigen Zübereitung auf nasses Filterpapier gelegt, wobei c Blattunterseite nach oben gerichtet ist. Nach Antrocknen der so behandelten Proben werden sec erwachsene Weibchen von Tetranychus urticae auf jede Blattscheibe gesetzt und für 3 Tage bei 25 °C u 16 h pro Tag bei Licht gehalten (vier Wiederholungen). Dann werden die toten und lebenden Weibch gezählt und entnommen. Gleichfalls werden die abgelegten Eier gezählt. Nach weiteren sieben Tag werden die lebenden Larven gezählt. Unter Bezug auf die unbehandelte Kontrolle wird die Gesamtwirku nach Abbott berechnet.

Die erfindungsgemäßen Verbindungen gemäß den Beispielen Nr. 2 und 5 zeigten eine 80 - 100 %i Gesamtwirkung.

**Anwendungsbeispiel C**

Wirkung kurativer Behandlung gegen Eier der Gemeinen Bohnenspinnmilbe (Tetranychus urticae Koch)

Aus entwickelten Primärblättern der Buschbohne (Phaseolus vulgaris nanus Aschers.) werden ru Blattscheiben mit einem Durchmesser von 14 mm gestanzt und mit abwärts gerichteter Oberseite a nasses Filterpapier gelegt. Je Blattscheibe werden mindestens 5 adulte Weibchen von Tetranychus urtic aufgesetzt und für 2 Tage bei etwa 25 °C, 50 - 60 % relativer Luftfeuchte und 16 Stunden Licht pro T gehalten. Nach dem Absammeln der Adulten werden die Blattscheiben mit den abgelegten Eiern in ei 0,016 % Wirkstoff und Netzmittel enthaltende Zubereitung getaucht. Als Kontrolle werden Blattscxheiben Wasser getaucht, wobei das Netzmittel in gleicher Konzentration wie in der Wirkstoff enthaltend Zubereitung enthalten ist. Nach Zählung der Eier werden die Blattscheiben für 7 Tage bei etwa 25 °C, 50 60 % relativer Luftfeuchte und 16 Stunden Licht pro Tag gehalten. Aus der prozentualen Differe abgelegter Eier und lebender Larven wird unter Bezug auf die Kontrolle nach Abbott die Wirkung berechn Der Durchschnitt wergibt sich aus drei Wiederholungen.

Eine Wirkung von 80 % oder mehr zeigte die erfindungsgemäße Verbindung gemäß Beispiel 4.

**Anwendungsbeispiel D**

Abtötende Wirkung auf Eier/Larven des Maiswurzelwurmes (Diabrotica undecimpunctata)

Die erfindungsgemäßen Verbindungen werden als wäßrige Zübereitung mit der Wirkstoffkonzentrati 0,1 % eingesetzt. Von diesen Wirkstoffzubereitungen werden 0,2 ml auf den Boden einer Polystyrolpet schale sowie auf den darin enthaltenden Maiskeimling und auf die im Schalenzentrum befindlichen ca. Eier des Maiswurzelwurmes (Diabrotica undecimpunctata) pipettiert. Die verschlossenen Schalen werden f 4 Tage bei 25 °C unter Langtagbedingungen aufgestellt. Kriterium für die Wirkungsbeurteilung ist c Abtötung von Eiern oder der frisch schlüpfenden Larven bei Versuchsende.

Die Verbindung gemäß Beispiel 5 erzielte eine 80 - 100 %ige Wirkung.

## Ansprüche

1. Verbindungen der allgemeinen Formel I

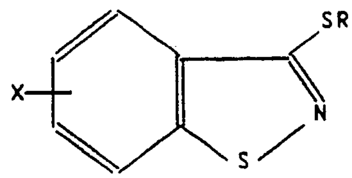

(I),

worin
X Wasserstoff, Halogen, $C_{1-4}$-Alkyl, $C_{1-4}$-Alkylthio, $C_{1-4}$-Alkoxy, Di-$C_{1-4}$-alkylamino, Halogen-$C_{1-4}$-alk· Halogen-$C_{1-4}$-alkoxy, Halogen-$C_{1-4}$-alkylthio, $C_{3-6}$-cycloalkyl, Halogen-$C_{3-6}$-cycloalkyl, Halogen-$C_{3-6}$-c cloalkylmethoxy, Halogen-$C_{3-6}$-Cycloalkylmethylthio, Nitro, Cyano, Amino, Phenyl, Halogenphenyl, Ph noxy, Phenylthio oder Halogenphenylthio
und
R einen ein- oder mehrfach, gleich oder verschieden durch Halogen substituierten $C_{1-12}$-Alkyl-, $C_{2-1}$ Alkenyl-, $C_{2-12}$-Alkinyl-, $C_{3-6}$-Cycloalkyl- oder $C_{3-6}$-Cycloalkylmethylrest bedeuten.

2. Verfahren zur Herstellung von Verbindungen der Formel I, dadurch gekennzeichnet, daß m Verbindungen der Formel II

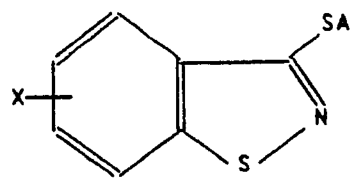

(II),

wobei X die in Formel I angegebene Bedeutung hat und A Wasserstoff, Ammonium oder ein Alkalimet bedeutet, mit Verbindungen der Formel Z-R, wobei Z eine Fluchtgruppe wie z.B. Halogen, Mesylat u Tosylat ist und R die in Formel I angegebene Bedeutung hat, in einem inerten Lösungsmittel od Lösungsmittelgemisch bei gebenenfalls erhöhter Temperatur und gegebenenfalls bei erhöhtem Druck Gegenwart einer Base umsetzt.

3. Verwendung von Verbindungen gemäß Anspruch 1 der Formel I zur Bekämpfung von Schädlinge insbesondere Nematoden.

4. Schädlingsbekämpfungsmittel gekennzeichnet durch einen Gehalt an mindestens einer Verbindu gemäß Anspruch 1 der Formel I.

5. Schädlingsbekämpfungsmittel gemäß Anspruch 4 in Mischung mit Träger-und/oder Hilfsstoffen.